# EUROPÄISCHE PATENTSCHRIFT

(11) **EP 3 216 486 B1**
(45) Veröffentlichungstag und Bekanntmachung des Hinweises auf die Patenterteilung: **27.01.2021**
(21) Anmeldenummer: 17157260.5
(22) Anmeldetag: 21.02.2017
(51) Int. Cl.: A61M 39/04, A61M 39/26, A61M 39/10, A61J 15/00

(54) **VERBINDUNGSEINRICHTUNG EINES MEDIZINISCHEN INFUSIONSSYSTEMS**
CONNECTING DEVICE OF A MEDICAL INFUSION SYSTEM
DISPOSITIF DE LIAISON D'UN SYSTÈME D'INFUSION MÉDICAL

(30) Priorität: 03.03.2016 DE 102016203518
(43) Veröffentlichungstag der Anmeldung: 13.09.2017
(73) Patentinhaber: B. Braun Melsungen AG, 34212 Melsungen (DE)
(72) Erfinder: BERTSCHI, Samuel, 6170 Schüpfheim (CH); DENK, Matthias, 3550 Langnau i.E. (CH); DUDHANE, Mayur, 6182 Escholzmatt (CH); EISEN, Frank, 6182 Escholzmatt (CH); KUNSCHAK, Ralf, 6130 Willisau (CH); SCHLITT, Christof, 34621 Obergrenzebach (DE); SCHNEIDER, Martin, 3604 Thun (CH); ZWYGART, Fritz, 3415 Hasle bei Burgdorf (CH)
(74) Vertreter: Patentanwälte Ruff, Wilhelm, Beier, Dauster & Partner mbB

(56) Entgegenhaltungen:
- WO-A1-99/24090
- WO-A2-03/066152
- US-A1- 2002 002 351
- US-A1- 2012 089 086

## Beschreibung

Die Erfindung betrifft eine Verbindungseinrichtung eines medizinischen Infusionssystems, mit einem Anschlussstutzen, der eine Verbindungsprofilierung zum Anschluss eines Funktionsteils des Infusionssystems aufweist, sowie mit einem elastisch nachgiebigen, becherartigen Ventilkörper, der in dem Anschlussstutzen angeordnet ist und einen Ventilmantel sowie einen deckelförmigen Kopfbereich aufweist, der mit einer Schlitzanordnung versehen ist, und mit einem formstabilen Bodenabschnitt, auf dem ein Bodenring des Ventilmantels abgestützt ist, und der mit dem Anschlussstutzen fest verbunden ist.

Eine derartige Verbindungseinrichtung eines medizinischen Infusionssystems ist aus der US 2012/0089086 A1 bekannt. Die bekannte Verbindungseinrichtung weist einen Anschlussstutzen auf, der mit einem Luer-Anschluss zum Ansetzen einer Spritze versehen ist. In dem Anschlussstutzen ist ein elastisch nachgiebiger, becherartiger Ventilkörper vorgesehen, der einen Ventilmantel sowie einen deckelförmigen Kopfbereich aufweist, in dem ein Schlitz vorgesehen ist. Der Ventilmantel des Ventilkörpers weist einen Bodenring auf, der in dem Anschlussstutzen fixiert ist.

Eine weitere Verbindungseinrichtung ist aus der EP 1 217 284 B1 allgemein bekannt. Die Verbindungseinrichtung weist einen Anschlussstutzen auf, in dem ein Ventilkörper integriert ist. Der Ventilkörper schließt den Anschlussstutzen in unbelastetem Ausgangszustand dicht ab und ist becherartig sowie elastisch nachgiebig ausgeführt. Der Ventilkörper ist einteilig aus einem Elastomermaterial hergestellt. Ein Kopfbereich des Ventilkörpers ist mit einer Schlitzanordnung versehen, die sich bei einer elastischen Deformation des Ventilkörpers aufweitet und so einen Durchfluss durch den Ventilkörper hindurch freigibt. Eine elastische Deformation des Ventilkörpers erfolgt beim Konnektieren des Anschlussstutzens mit einem weiteren Funktionsteil des medizinischen Infusionssystems, insbesondere einer Infusionsspritze, einem Schlauchsystem oder Ähnlichem.

Die EP 1 470 352 B1 zeigt eine weitere Verbindungseinrichtung eines medizinischen Infusionssystems, bei dem ebenfalls in einem Anschlussstutzen ein elastisch deformierbarer Ventilkörper integriert ist, der becherartig gestaltet ist und in einem deckelförmigen Kopfbereich eine öffnungsfähige Schlitzanordnung aufweist.

Eine weitere Verbindungseinrichtung ist aus der WO 2013/017698 A1 bekannt. Die bekannte Verbindungseinrichtung weist einen Anschlussstutzen auf, der mit Luer-Profilierungen versehen ist, um das Konnektieren eines mit einem komplementären Luer-Anschluss versehenen Funktionsteils des medizinischen Infusionssystems zu ermöglichen. In dem Anschlussstutzen ist zur Abdichtung einer Öffnung des Anschlussstutzens ein becherartiger Ventilkörper integriert, der einstückig aus einem elastisch deformierbaren Material hergestellt ist. Der Ventilkörper erweitert sich ausgehend von einem deckelförmigen Kopfbereich zu einem Bodenring hin im Wesentlichen stetig, wodurch sich eine Glockenform für den Ventilkörper ergibt. Der Ventilkörper ist in gleicher Weise mit einer Schlitzanordnung in seinem Kopfbereich versehen, wie dies bei den Ventilkörpern der zuvor beschriebenen Verbindungseinrichtungen der Fall ist.

Aufgabe der Erfindung ist es, eine Verbindungseinrichtung der eingangs genannten Art zu schaffen, die ein sicheres Konnektieren und Dekonnektieren eines Funktionsteiles ermöglicht, ohne dass ein Fluidverlust oder eine Fluidkontamination auftritt.

Diese Aufgabe wird durch die Merkmale des Anspruchs 1 gelöst. Hierdurch ist ein einfaches Öffnen des Ventilkörpers durch elastische Deformation sowie ein zuverlässiges Rückstellen des Ventilkörpers in eine unbelastete, geschlossene Ausgangslage ermöglicht. Die erfindungsgemäße Lösung eignet sich in besonders vorteilhafter Weise für den Einsatz in einer Verbindungseinrichtung in Form eines Dreiwegehahns. Die feste Verbindung des formstabilen Bodenabschnittes mit dem Anschlussstutzen kann durch eine lösbare oder unlösbare Verbindung vorgesehen sein. Alternativ können der Anschlussstutzen und der formstabile Bodenabschnitt einstückig miteinander verbunden sein. Der Anschlussstutzen kann als formstabile Kappe ausgeführt sein, der fest mit dem Bodenabschnitt verbindbar ist und den Ventilkörper aufnimmt sowie relativ zum Bodenabschnitt fixiert. Eine Fixierung des Ventilkörpers mittels seines Bodenringes im Bereich des formstabilen Bodenabschnittes kann kraftschlüssig durch Verklemmung und/oder stoffschlüssig durch Verklebung oder Verschweißung vorgesehen sein. Die Schlitzanordnung des Kopfbereichs des Ventilkörpers ist entweder als geschlossener Schlitz, insbesondere in Form eines Einschnitts, oder aber nach Art eines teilweise offenen Spaltes ausgeführt. Der Begriff der Schlitzanordnung umfasst demzufolge sowohl eine geschlossene als auch eine teilweise offene Gestaltung.

In Ausgestaltung der Erfindung verdickt eine Wandung des Ventilmantels sich ausgehend von dem Kopfbereich zum Bodenring hin. Hierdurch ist eine sich vom Kopfbereich zum Bodenring hin reduzierende elastische Flexibilität gegeben. Die Verdickung ist unstetig und nichtlinear ausgeführt.

In weiterer Ausgestaltung der Erfindung ist eine Aufstandsfläche des Bodenrings zumindest abschnittsweise konisch gestaltet, und der formstabile Bodenabschnitt ist komplementär konisch gestaltet für eine flächige Abstützung des Bodenrings auf dem Bodenabschnitt. Dadurch wird eine sichere Zentrierung und Fixierung des Bodenringes des Ventilkörpers am formstabilen Bodenabschnitt erreicht. Hierdurch ist insbesondere eine vereinfachte Montage des Ventilkörpers im Anschlussstutzen und am Bodenabschnitt erzielbar.

In weiterer Ausgestaltung der Erfindung ist eine Außenfläche des deckelförmigen Kopfbereichs eben gestaltet und schließt bündig mit einer Randkante des Anschlussstutzens ab. Eine Randkante des Anschlussstutzens ist insbesondere eine Anfasung des Randbereichs des Anschlussstutzens. Alternativ kann unter der Randkante des Anschlussstutzens auch eine Außenrandfläche des Anschlussstutzens verstanden werden. Durch die ebene Gestaltung der Außenfläche ist ein einfaches Reinigen und Desinfizieren des Kopfbereichs und des Randbereichs des Anschlussstutzens durch einfaches Abwischen oder Abtupfen mittels eines Desinfektionstuches erzielbar. Dies erleichtert die Handhabbarkeit für das medizinische Personal. Die glatten Flächen verhindern zudem Kontaminationen der Außenfläche des Kopfbereichs oder des Randbereichs des Anschlussstutzens.

In weiterer Ausgestaltung der Erfindung ist eine nach innen weisende Kontur des deckelförmigen Kopfbereichs domartig gestaltet. Die domartige Gestaltung kann als konvexe Bombierung oder auch als polygonale oder kegelförmige Spitze gestaltet sein. Hierdurch wird der Kopfbereich verdickt, wodurch eine verbesserte und beschädigungsfreie Einbringung der Schlitzanordnung in den Kopfbereich erzielbar ist.

Die der Erfindung zugrunde liegende Aufgabe wird auch dadurch gelöst, dass der formstabile Bodenabschnitt eine ringförmige Gegenschulter aufweist, die in den Bodenring des Ventilmantels in Richtung des Kopfbereichs hineinragt. Die ringförmige Gegenschulter dient dazu, ein Eintauchen einer Spitze eines Funktionsteiles beim Konnektieren dieses Funktionsteiles mit dem Anschlussstutzen in den Anschlussstutzen hinein und demzufolge in den Ventilkörper hinein zu begrenzen. Durch die Begrenzung der Eintauchtiefe wird eine Überlastung der elastischen Deformation des Ventilkörpers vermieden, woraus sich eine längere Haltbarkeit des Ventilkörpers ergibt. Zudem werden hohe Lösekräfte bei einem Dekonnektieren des Funktionsteils vermieden. Das anzuschließende Funktionsteil weist vorzugsweise eine männliche Luer-Spitze eines Luer-Slip- oder eines Luer-Lock-Anschlusses auf, die in den Anschlussstutzen unter elastischer Deformation des Ventilkörpers eintaucht.

In Ausgestaltung der Erfindung weist die Gegenschulter eine ebene, radial zu einer Mittellängsachse des Anschlussstutzens ausgerichtete Stirnfläche auf. Dadurch trifft das mit dem Anschlussstutzen konnektierte Funktionsteil flächig und auf Stoß auf die Gegenschulter auf, wodurch sich eine besonders zuverlässige und genaue Begrenzung der Eindringtiefe des Funktionsteiles ergibt.

In weiterer Ausgestaltung der Erfindung weist der Bodenabschnitt eine die Gegenschulter umgebende Ringnut auf, in die der Ventilmantel bei einer elastischen Deformation teilweise eintaucht, und die in unbelastetem Ausgangszustand des Ventilkörpers einen freien Ringraum bildet. Der so gebildete Freiraum ermöglicht ein elastisches Ausweichen des Materials des Ventilmantels, so dass eine axiale Kompression des Ventilkörpers erzielbar ist. Die axiale Kompression definiert zudem eine axiale Vorspannung, die gewährleistet, dass der Ventilkörper nach dem Dekonnektieren des Funktionsteiles wieder zuverlässig in seine unbelastete Ausgangslage zurückkehrt, in der der Anschlussstutzen wieder dicht verschlossen ist.

In weiterer Ausgestaltung der Erfindung weist die Ringnut eine gestufte Ringprofilierung auf. Die gestufte Ringprofilierung ist vorzugsweise einer komplementären Stufung einer Innenkontur des Bodenringes des Ventilkörpers nachgestaltet, um eine verbesserte axiale Kompression in Form eines stufenartigen Kraftaufbaus zu definieren. Der stufenartige Kraftaufbau ergibt sich dadurch, dass bei einer axialen Deformation sich die komplementären Ringstufen bis zu einer definierten Krafterhöhung kurzfristig aneinander abstützen.

Die der Erfindung zugrunde liegende Aufgabe wird auch dadurch gelöst, dass eine Innenkontur des Ventilmantels in Abstand zu einer Innenfläche des Kopfbereichs mit einer radial nach außen erstreckten Ringausnehmung versehen ist, die insbesondere ein Festkörpergelenk für den Ventilmantel bei einer elastischen Deformation des Ventilkörpers definiert. Die Ringausnehmung bildet einen radialen ringförmigen Freiraum, in die der Kopfbereich bei einer elastischen Deformation und einem Einknicken nach innen eintauchen kann, während im Inneren des Ventilkanals mehr Raum für das Eindringen der Spritze des Festkörperteils zur Verfügung steht. Hierdurch ist zudem bei einer axialen Kompression des Ventilkörpers ein ringförmiges Einknicken, das heißt Einstülpen, des Kopfbereichs zum Bodenring hin nach innen gewährleistet, wodurch die Schlitzanordnung sich aufweitet und entsprechende Außenflächenbereiche des Kopfbereichs und des Ventilmantels sich an eine entsprechend eindringende Luer-Spitze des Funktionsteils flächig anlegen. Hierdurch ergibt sich eine besonders gute Abdichtung zwischen Ventilkörper und Funktionsteil. Bei einem Dekonnektieren und demzufolge einem axialen Herausziehen der Luer-Spitze werden zwangsläufig die nach innen gestülpten Abschnitte des Ventilkörpers wieder nach außen mitgenommen, so dass der Ventilkörper sich wieder in seine Ausgangslage zurückstellt, in der er den Anschlussstutzen dicht abschließt.

In Ausgestaltung der Erfindung weist der Bodenring eine Ringschulter mit einem verdünnten Wandungsbereich auf, der derart gestaltet ist, dass bei einer axialen Druckbeanspruchung des Ventilkörpers vom Kopfbereich her ein axiales Einstülpen des Ventilmantels im Bereich des Bodenringes erfolgt. Dadurch ergibt sich eine klar definierte axiale Deformation des Ventilkörpers, die eine sichere Rückstellung des Ventilkörpers in eine unbelastete Ausgangslage ermöglicht, ohne dass die Gefahr einer dauerhaften Knickstelle und demzufolge Fehlfunktion des Ventilkörpers entsteht.

In weiterer Ausgestaltung der Erfindung ist der Ventilmantel rotationssymmetrisch und der Kopfbereich rotationsunsymmetrisch, insbesondere oval, gestaltet. In vorteilhafter Weise ist auch ein Öffnungsbereich des Anschlussstutzens, der mit seinem Randbereich den Kopfbereich des Ventilkörpers in dessen unbelasteter Ausgangslage umgibt, komplementär oval gestaltet.

In weiterer Ausgestaltung der Erfindung ist die Schlitzanordnung entlang einer Querstreckung des Kopfbereichs eingebracht. Dies ermöglicht ein sicheres Öffnen und Schließen der Schlitzanordnung bei einer entsprechenden elastischen Deformation.

Die Erfindung betrifft auch einen Ventilkörper für eine Verbindungseinrichtung, wie sie zuvor beschrieben wurde, wobei der Ventilkörper elastisch nachgiebig und becherartig gestaltet ist und mit einem Kopfbereich sowie einem Ventilmantel versehen ist, wie sie anhand der zuvor beschriebenen Merkmale ausgeführt sind.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in einer Draufsicht eine Ausführungsform einer erfindungsgemäßen Verbindungseinrichtung in Form eines Dreiwegehahns,
- Fig. 2: einen Schnitt durch die Verbindungseinrichtung nach Fig. 1 entlang der Schnittlinie II-II in Fig. 1,
- Fig. 3: eine Ansicht von unten eines Ventilkörpers der Verbindungseinrichtung gemäß Fig. 2,
- Fig. 4: einen Längsschnitt durch den Ventilkörper nach Fig. 3 entlang der Schnittlinie IV-IV in Fig. 3,
- Fig. 5: einen weiteren Längsschnitt durch den Ventilkörper längs der Schnittlinie V-V in Fig. 3,
- Fig. 6: eine Draufsicht auf den Ventilkörper nach den Fig. 3 bis 5,
- Fig. 7 bis 9: in Schnittdarstellungen verschiedene Schritte bei einer Konnektierung eines Funktionsteils an einen Anschlussstutzen der Verbindungseinrichtung nach Fig. 2,
- Fig. 10 bis 12: in verschiedenen Funktionsschritten eine weitere Konnektierung eines Funktionsteils an einen Anschlussstutzen einer anderen Ausführungsform einer erfindungsgemäßen Verbindungseinrichtung ähnlich Fig. 2,
- Fig. 13 bis 16: in Schnittdarstellungen verschiedene Funktionsschritte einer Konnektierung eines Funktionsteils an einen Anschlussstutzen einer weiteren Ausführungsform einer erfindungsgemäßen Verbindungseinrichtung, und
- Fig. 17 bis 20: in Schnittdarstellungen Funktionsschritte der Verbindung eines Funktionsteils an einen Anschlussstutzen einer weiteren Ausführungsform einer erfindungsgemäßen Verbindungseinrichtung.

Eine Verbindungseinrichtung 1 eines medizinischen Infusionssystems ist als Dreiwegehahn gestaltet. Die Verbindungseinrichtung 1 weist ein Gehäuse 2 auf, in dem ein Stellglied 6 drehbeweglich gelagert ist. In dem Gehäuse 2 sind insgesamt drei Verbindungskanäle vorgesehen, die je nach Stellung des Stellgliedes 6 abgesperrt oder miteinander verbunden werden. Ein Verbindungskanal des Gehäuses 2 führt zu einem Anschlussstutzen 3. Ein weiterer, rechtwinklig angeordneter Verbindungskanal führt zu einem Anschlussbereich 4 und gegenüberliegend hierzu ein dritter Verbindungskanal zu einem Anschlussbereich 5. Einer der beiden Anschlussbereiche 4, 5 ist für den Anschluss einer Patientenleitung vorgesehen. Der andere Anschlussbereich 4, 5 dient dazu, eine Verbindungsleitung zu einem Fluidbehältnis anzuschließen.

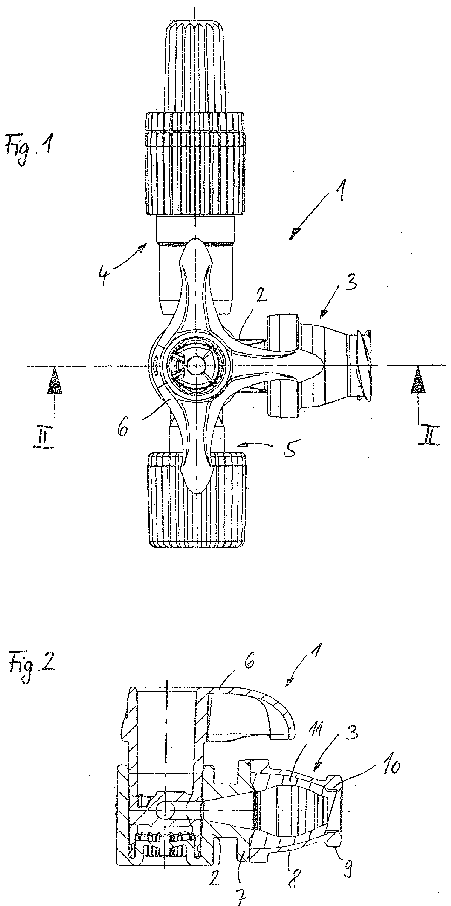

Der Anschlussstutzen 3 ist zum zeitweisen Konnektieren eines Funktionsteiles des Infusionssystems, wie insbesondere einer Spritze, vorgesehen, um der Patientenleitung zusätzliche Medikamente oder Ähnliches zuzuführen. Der Anschlussstutzen 3 ist anhand der Fig. 2 bis 9 näher erläutert.

Der Anschlussstutzen 3 weist eine formstabile Kappe 8 auf, die an ihrer dem Gehäuse 2 zugewandten Stirnseite mit einem formstabilen Bodenabschnitt 7 des Gehäuses 2 fest verbunden ist. Die Kappe 8 ist hülsenartig gestaltet und weist auf der dem Bodenabschnitt 7 zugewandten Seite einen verdickten Randbereich auf, der mit dem Bodenabschnitt 7, vorliegend durch Verschweißung, fest verbunden ist. Der Bodenabschnitt 7 ist tellerartig ausgeführt und ragt relativ zu einer Mittellängsachse L des Anschlussstutzens 3 radial nach außen ab. Der Bodenabschnitt 7 umgibt einen sich zu einem Inneren des Gehäuses 2 konisch verjüngenden Kanalabschnitt.

Die Kappe 8 ist an ihrem zu dem Bodenabschnitt 7 abliegenden Stirnbereich mit einem Durchtritt 10 versehen, der durch einen nachfolgend näher beschriebenen Ventilkörper 11 verschließbar ist. Der Durchtritt 10 wird von einem verdickten Randbereich umschlossen, der mit Verbindungsprofilierungen 9 in Form von Luer-Lock-Profilierungen versehen ist.

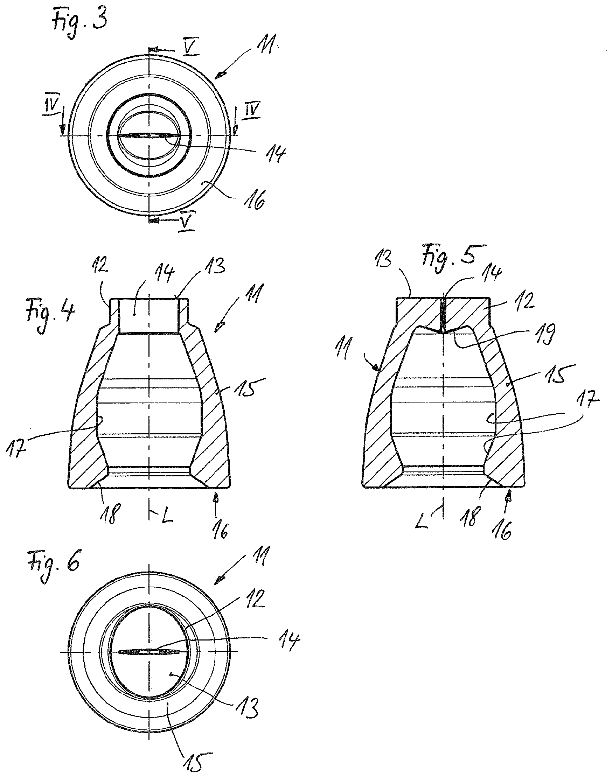

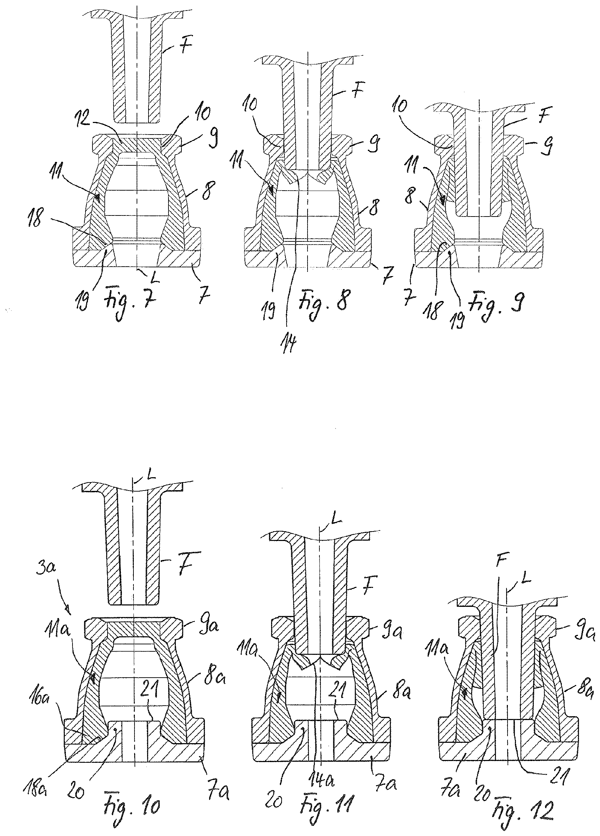

Der Ventilkörper 11 ist becher- oder glockenartig gestaltet und einstückig aus einem elastisch nachgiebigen Werkstoff hergestellt, vorliegend aus einem Elastomer oder einem thermoplastischen Elastomer. Besonders vorteilhaft ist der Ventilkörper 11 aus Silikon hergestellt. Der Ventilkörper 11 weist eine Außenkontur auf, die in unbelastetem Ausgangszustand über eine gesamte Höhe der Kappe 8 bündig und flächig an der Innenkontur der Kappe 8 anliegt. Der Ventilkörper 11 ist mit einem deckelförmigen Kopfbereich 12 versehen, der eine rotationsunsymmetrische, vorliegend eine ovale, Grundfläche aufweist (siehe insbesondere Fig. 3 und 6). An den Kopfbereich 12 schließt ein Ventilmantel 15 an, der an einem unteren Stirnendbereich mit einem Bodenring 16 versehen ist. Der Kopfbereich 12 ist mit einer Schlitzanordnung 14 versehen. Eine Oberfläche 13 des Kopfbereichs 12 ist glatt und eben gestaltet. Anhand der Fig. 7 ist erkennbar, dass die Oberfläche 13 des Kopfbereichs 12 in unbelastetem Ausgangszustand des Ventilkörpers 11 in der Kappe 8 mit einer Randkante des Durchtrittes 10 bündig abschließt. Von dieser Randkante des Durchtrittes 10 aus erstreckt sich eine schräge Anfasung nach außen bis zu einer Stirnfläche des den Durchtritt 10 definierenden Randbereichs der Kappe 8. In der unbelasteten Ausgangslage des Ventilkörpers 11 kann demzufolge die Stirnfläche des Randbereichs der Kappe 8 einschließlich der Oberfläche des Kopfbereichs 12 des Ventilkörpers 11 in einfacher Weise mittels eines Desinfektionstuchs oder Ähnlichem durch medizinisches Personal gereinigt und desinfiziert werden.

Der Ventilmantel 15 des Ventilkörpers 11 ist relativ zu der Mittellängsachse L rotationssymmetrisch ausgeführt und weist eine Wandung auf, die sich ausgehend von dem Kopfbereich 12 bis zu dem Bodenbereich 16 hin verdickt. Die Verdickung erfolgt unstetig und nichtlinear, wie anhand der beiden dargestellten sichtbaren Kanten erkennbar ist. Die Kanten sind ringförmig umlaufend. Dabei weist der Ventilmantel 15 einen an den Kopfbereich 12 anschließenden, sich kegelstumpfartig erweiternden ersten Wandungsabschnitt mit gleichbleibender Dicke auf. An diesen ersten Wandungsabschnitt schließt in Richtung des Bodenringes 16 ein zweiter Wandungsabschnitt an, dessen Innenwandung zylindrisch und koaxial zur Mittellängsachse L verläuft, und dessen Außenwandung zum Bodenring 16 hin weiter nach außen gewölbt verläuft. An diesen mittleren Wandungsabschnitt schließt sich der bodenseitige Wandungsabschnitt an, der den Bodenring 16 umfasst. In diesem Bereich verläuft die Innenwandung ausgehend von dem zylindrischen Mittelbereich zum Bodenabschnitt hin verjüngt zu, wodurch sich ein konisch nach unten verjüngt zulaufender Innenwandungsabschnitt ergibt.

Zu einer Stirnseite des Bodenringes 16 hin erweitert sich die Innenwandung wieder konisch unter Bildung einer Aufstandsfläche 18. Über die Höhe des Ventilmantels 15 ergibt sich demzufolge eine eiartige oder O-artige Innenkontur 17 (Fig. 4 und 5).

Eine ins Innere des Ventilmantels 15 weisende Innenfläche des Kopfbereichs 12 ist als domartige Kontur 19 gestaltet, wie Fig. 5 zu entnehmen ist.

Der Durchtritt 10 der Kappe 8 kann rotationssymmetrisch oder rotationsunsymmetrisch gestaltet sein. Bei rotationsunsymmetrischem Kopfbereich 12 ist vorzugsweise auch der Durchtritt 10 in komplementärer Weise rotationsunsymmetrisch gestaltet.

Wie anhand der Fig. 2 und 7 bis 9 erkennbar ist, ist der konischen Aufstandsfläche 18 des Bodenrings 16 des Ventilkörpers 11 im Bereich des Bodenabschnitts 7 eine komplementär konische Stützfläche 19 zugeordnet, wodurch der Ventilkörper 11 sich im Bereich des Bodenringes 16 über seine gesamte radiale Breite flächig auf dem Bodenabschnitt 7 abstützt.

Die Schlitzanordnung 14 ist quer zu einer Längserstreckung des ovalen Kopfbereichs 12 ausgerichtet, wie anhand der Fig. 4 bis 6 erkennbar ist. Die Schlitzanordnung 14 erstreckt sich mittig längs der Mittellängsachse L durch den Kopfbereich 12 hindurch.

Sobald nun eine Spitze eines mit dem Anschlussstutzen 3 zu konnektierenden Funktionsteils F(siehe Fig. 7 bis 9) von außen her an den Anschlussstutzen 3 herangeführt wird, kommt die Spitze flächig mit der Außenfläche des Kopfbereichs 12 in Kontakt und drückt den Kopfbereich 12 in das Innere der Kappe 8 hinein. Dabei weitet sich die Schlitzanordnung 14, und die elastisch deformierten Abschnitte des Kopfbereichs 12 legen sich bei einem weiteren Eindringen der Spitze des Funktionsteils F außen an die Spitze an. Bei einem Dekonnektieren und einem daraus resultierenden Entfernen der Spitze nach außen stellt sich der Kopfbereich 12 wieder in die Ausgangslage gemäß Fig. 7 zurück.

Die Verbindungseinrichtung gemäß den Fig. 10 bis 12 entspricht im Wesentlichen der zuvor beschriebenen Verbindungseinrichtung gemäß den Fig. 1 bis 9. Funktionsgleiche Teile und Abschnitte sind mit gleichen Bezugszeichen unter Hinzufügung des Buchstabens a versehen. Zur Vermeidung von Wiederholungen wird auf die Offenbarung zu der Ausführungsform nach den Fig. 1 bis 9 verwiesen. Der Anschlussstutzen 3a entspricht im Wesentlichen dem Anschlussstutzen 3 der Verbindungseinrichtung gemäß den Fig. 1 bis 9. Die Kappe 8a wie auch der Ventilkörper 11a sind identisch wie die Kappe 8 und der Ventilkörper 11 gemäß den Fig. 1 bis 9 gestaltet. Die Kappe 8a kann insbesondere durch Verschweißung stoffschlüssig mit dem Bodenabschnitt 7a verbunden sein. Besonders vorteilhaft ist die Kappe 8a lösbar mit dem Bodenabschnitt 7a des Gehäuses verbunden, insbesondere durch Verschraubung. Hierdurch ist ein Austausch des Ventilkörpers 11a in einfacher Weise ermöglicht.

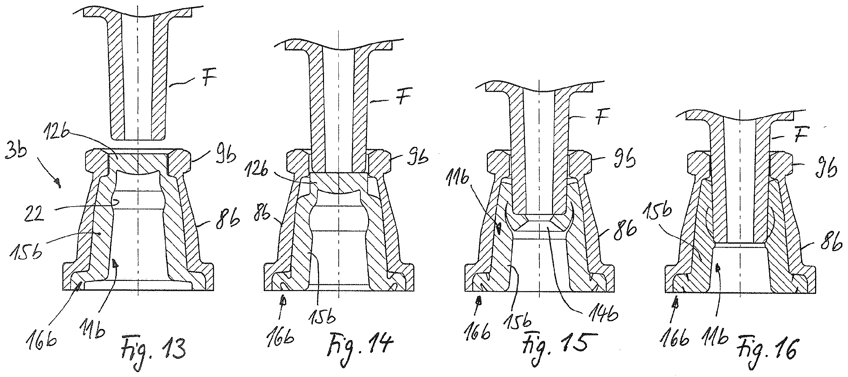

Auch die Kappe 8 sowie die nachfolgend beschriebenen Kappen 8b und 8c der Ausführungsformen nach den Fig. 13 bis 20 können - je nach Ausführung - lösbar oder unlösbar mit dem jeweiligen Bodenabschnitt 7, 7b, 7c verbunden sein. Die lösbare Verbindung erfolgt vorteilhaft durch eine Verschraubung.

Wesentlicher Unterschied des Bodenabschnittes 7a zu dem Bodenabschnitt 7 ist es, dass der Bodenabschnitt 7a im Anschluss an die konische Abstützfläche 18a für den Bodenring 16a des Ventilkörpers 11a noch mit einer einstückig angeformten, ringförmigen Gegenschulter 20 versehen ist, die zylinderartig zu dem Kopfbereich des Ventilkörpers 11a hin in die Innenkontur des Ventilkörpers 11a hineinragt. Die Gegenschulter 20 ist mit einer ebenen und relativ zur Mittellängsachse L radial erstreckten Stirnfläche 21 versehen. Die Gegenschulter 20 dient als Anschlag für die Spitze des Funktionsteils F, so dass eine Eindringtiefe der Spitze des Funktionsteils in den Anschlussstutzen 3a begrenzt ist. Die Spitze des Funktionsteils F ist mit einer komplementären ebenen Stirnfläche versehen, die in konnektiertem Zustand des Funktionsteils radial zur Mittellängsachse L erstreckt ist und demzufolge flächig und bündig auf der Stirnfläche 21 der Gegenschulter 20 aufliegt (Fig. 12).

Dadurch, dass die Gegenschulter 20 die Eindringtiefe der Spitze begrenzt, ist zum einen gewährleistet, dass der Ventilkörper 11a nicht zu stark deformiert wird, wodurch Beschädigungen des Ventilkörpers 11a auftreten könnten. Zum anderen gewährleistet die Begrenzung der Eindringtiefe der Spitze, dass die Spitze sich nicht im Bereich des Durchtrittes des Anschlussstutzens 3a verkeilt, so dass eine Dekonnektierung des Funktionsteils einschließlich der Spitze ohne großen Kraftaufwand ermöglicht ist.

Die Ventileinrichtung gemäß den Fig. 13 bis 16 weist einen Anschlussstutzen 3b auf, der in nicht näher dargestellter Weise auf einem Bodenabschnitt des Gehäuses befestigt ist. Funktionsgleiche Teile und Abschnitte der Verbindungseinrichtung bzw. des Anschlussstutzens 3b sind mit gleichen Bezugszeichen unter Hinzufügung des Buchstabens b versehen. Auch bei dieser Ausführungsform wird bezüglich der funktionsgleichen Teile und Abschnitte zur Vermeidung von Wiederholungen auf die zuvor beschriebenen Ausführungsformen verwiesen. Nachfolgend wird auf die Unterschiede des Anschlussstutzens 3b eingegangen.

Der Ventilkörper 11b ist in gleicher Weise als einstückiger Elastomerkörper gestaltet, wie die Ventilkörper 11 und 11a gemäß den zuvor beschriebenen Ausführungsformen. Der Ventilkörper 11b weist jedoch eine unterschiedliche Form und eine unterschiedliche Deformationsfunktion auf. Der becher- oder glockenartige Ventilkörper 11b ist mit einem Kopfbereich 12b versehen, an den sich ein Ventilmantel 15b anschließt, der in einen Bodenring 16b übergeht. Der Kopfbereich 12b ist mit einer Schlitzanordnung 14b versehen. Der Ventilmantel 15b ist in Abstand unterhalb des Kopfbereichs 12b im Bereich seiner Innenkontur mit einer ringförmigen Ausnehmung 22 versehen, die im Ventilmantel ein ringförmiges Festkörpergelenk bildet. Zudem ist am Übergang des Ventilmantels 15b zu dem Bodenring 16b eine radial nach außen erstreckte Ringstufe vorgesehen, deren Wandungsstärke geringer ist als die Wandungsstärke des Ventilmantels 15b. Hierdurch wird ein weiteres, ringförmiges Festkörpergelenk gebildet. Schließlich geht der Ventilmantel 15b zum Kopfbereich 12b hin ebenfalls mit einem schmaleren Ringmantelbereich in den Kopfbereich 12b über. Auch in diesem Bereich wird demzufolge zwangsläufig ein ringförmiges Festkörpergelenk gebildet. Im Bereich der beschriebenen Festkörpergelenke ergeben sich demzufolge Faltungen oder Einstülpungen, sobald auf den Ventilkörper 11b eine Axialkraft von außen her ausgeübt wird. Dabei sind die Festkörpergelenke derart aufeinander abgestimmt, dass bei einer axialen Druckbelastung von außen durch eine Spitze eines Funktionsteiles F auf den Kopfbereich 12b zunächst der Kopfbereich 12b und der Ventilmantel 15b im Wesentlichen undeformiert axial nach innen gedrückt werden unter Verformung des Festkörpergelenks im Bereich des Bodenrings 16b (Fig. 14). Dabei kommt ein entsprechender Abschnitt des Ventilmantels 15b lediglich in angedeuteter Weise auf einer Stirnfläche des Bodenabschnittes des Gehäuses zur Anlage, so dass der Ventilmantel 15b nicht weiter axial nach innen gepresst werden kann. Das deformierte Festkörpergelenk im Bereich des Bodenringes 16b übt zwangsläufig eine axiale Gegenspannung auf den Ventilkörper 15b aus, die nach Wegnahme der Druckbelastung eine elastische Rückstellung des Ventilkörpers 11b in die unbelastete Ausgangslage gemäß Fig. 13 bewirkt. Wenn nun - wie anhand der Fig. 14 bis 16 erkennbar - die Spitze des Funktionsteils F weiter nach innen in den Anschlussstutzen 3b eindringt, kollabiert nun das Festkörpergelenk im Bereich des Kopfbereichs 12b, wobei die schmalere Ringstufe am Kopfbereich 12b elastisch deformiert wird. Eine Innenseite des Kopfbereichs 12b legt sich durch das Aufweiten der Stützanordnung 14b zwangsläufig im Bereich der Ausnehmung 22 an die Innenkontur des Ventilmantels 15b an, wodurch der Kopfbereich 12b nach innen und nach unten gedrückt wird. Der Ventilmantel 15b wird gegen die Innenwandung der Kappe 8b verdrängt, wodurch der Ventilmantel einschließlich des Kopfbereichs 12b sich im Wesentlichen flächig an die Außenkontur der Spitze F anlegt.

Bei einem Herausziehen der Spitze F stellt sich der Ventilkörper 15b wieder in die unbelastete Ausgangslage gemäß Fig. 13 zurück.

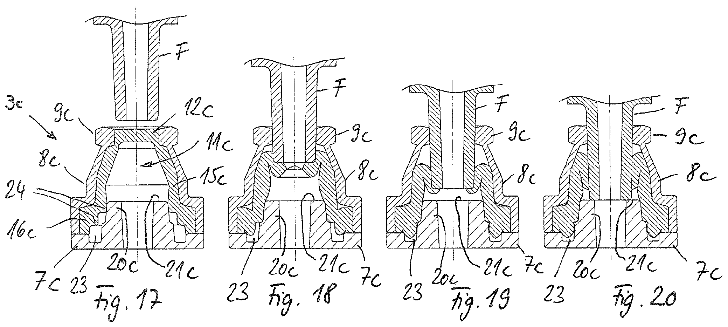

Der Anschlussstutzen 3c nach den Fig. 17 bis 20 ist ebenfalls bei einer Verbindungseinrichtung vorgesehen, wie sie anhand der Fig. 1 und 2 dargestellt ist. Funktionsgleiche Teile und Abschnitte sind mit gleichen Bezugszeichen unter Hinzufügung des Buchstabens c versehen. Zur Vermeidung von Wiederholungen wird ergänzend auf die Offenbarung zu den zuvor beschriebenen Ausführungsformen verwiesen. Auch der Anschlussstutzen 3c weist eine Kappe 8c auf, die mit einem Bodenabschnitt 7c des Gehäuses lösbar oder unlösbar fest verbunden ist. Die Kappe 8c weist an ihrem von dem Bodenabschnitt 7c abgewandten Stirnendbereich einen Durchtritt auf, der von einem Randbereich umschlossen ist, der mit Verbindungsprofilierungen 9c, vorliegend in Form von Luer-Lock-Profilierungen, versehen ist. In dem Anschlussstutzen 3c ist ein Ventilkörper 11c integriert, der als einstückiges Elastomerbauteil gestaltet ist. Der Ventilkörper 11c weist einen Kopfbereich 12c auf, der den Durchtritt der Kappe 8c verschließt, und der mit einer Schlitzanordnung versehen ist. Der Bodenabschnitt 7c ist mit einer Gegenschulter 20c zur Begrenzung der Eindringtiefe der Spitze eines als männliches Luer-Lock-Teil ausgeführten Funktionsteiles F versehen. Die Gegenschulter 20c weist eine Stirnfläche 21c auf, auf der die Stirnfläche der Spitze in konnektiertem Zustand aufliegt. Der Ventilkörper 11c ist mit einem ringförmigen Festkörpergelenk am Übergang des Ventilmantels 15c zum Bodenring 16c versehen. Der Bodenring 16c ist im Bereich seiner Innenseite mit zwei Ringstufen 24 versehen, die derart gestaltet sind, dass der Ventilmantel 15c bei einer axialen Druckbelastung durch die Spitze des Funktionsteils F sowohl im Bereich des Bodenrings 16c als auch am Übergang zwischen Kopfbereich 12c und Ventilkörper 15c ringförmig einknicken und demzufolge elastisch deformiert werden kann. Um das Einstülpen oder Einknicken im Bereich des Bodenringes 16c nach innen nicht zu behindern, ist am Übergang zwischen der Gegenschulter 20c und einer radialen Ringschulter des Bodenabschnitts 7c eine gestufte Ringnut 23 vorgesehen, die einen ringförmigen Freiraum bildet. Die gestufte Ringnut 23 erstreckt sich axial in den Bodenabschnitt 7c hinein und weist in Abstand oberhalb ihres Grundes einen nicht näher bezeichneten Ringabsatz auf, auf der bei einer axialen Deformation des Ventilkörpers 11c eine erste Ringstufe 24 des Ventilmantels 15c axial auftrifft. Bei weiterer axialer Deformation deformiert sich der Ventilkörper 11c am Übergang des Ventilmantels 15c zum Bodenring 16c weiter, wodurch das verdrängte Material des Ventilmantels 15c in den Freiraum 23 eintaucht, wie anhand der Fig. 19 und 20 erkennbar ist. Durch die Aufweitung der Schlitzanordnung kann die Spitze des Funktionsteils F auf die Stirnfläche 21c der Gegenschulter 20c auftreffen. Der Kopfbereich 12c legt sich außenseitig an die Spitze an, wie der Fig. 20 entnehmbar ist. Bei einem Dekonnektierendes Funktionsteils F wird die Spitze wieder aus dem Durchtritt entfernt, wodurch der Ventilkörper 11c sich wieder in die unbelastete Ausgangslage gemäß Fig. 17 zurückstellt.

## Patentansprüche

1. Verbindungseinrichtung eines medizinischen Infusionssystems, mit einem Anschlussstutzen (3), der eine Verbindungsprofilierung (9) zum Anschluss eines Funktionsteils (F) des Infusionssystems aufweist, sowie mit einem elastisch nachgiebigen, becherartigen Ventilkörper (11), der in dem Anschlussstutzen (3) angeordnet ist und einen Ventilmantel (15) sowie einen deckelförmigen Kopfbereich (12) aufweist, der mit einer Schlitzanordnung (14) versehen ist, und mit einem formstabilen Bodenabschnitt (7), auf dem ein Bodenring (16) des Ventilmantels (15) abgestützt ist, und der mit dem Anschlussstutzen (3) fest verbunden ist, **dadurch gekennzeichnet, dass** der Ventilmantel (15) in unbelastetem Ausgangszustand eine bombierte Innenkontur aufweist, die sich ausgehend von dem deckelförmigen Kopfbereich (12) in Richtung des Bodenringes (16) zunächst erweitert und sich anschließend wieder verjüngt unter Bildung eines O-artigen Innenlängsschnitts, wobei der Ventilmantel (15) einen an den Kopfbereich (12) anschließenden, sich kegelstumpfartig erweiternden ersten Wandungsabschnitt mit gleichbleibender Dicke aufweist, an den in Richtung des Bodenringes (16) ein mittlerer Wandungsabschnitt anschließt, dessen Innenwandung zylindrisch und koaxial zu einer Mittellängsachse L verläuft, und dessen Außenwandung zu dem Bodenring (16) hin weiter nach außen gewölbt verläuft, und wobei an den mittleren Wandungsabschnitt ein bodenseitiger Wandungsabschnitt anschließt, der den Bodenring (16) umfasst, und in dem die Innenwandung ausgehend von dem mittleren Wandungsabschnitt zum Bodenabschnitt konisch nach unten verjüngt zuläuft.

2. Verbindungseinrichtung nach Anspruch 1, **dadurch gekennzeichnet, dass** eine Wandung des Ventilmantels (15) sich ausgehend von dem Kopfbereich (12) zum Bodenring (16) hin verdickt.

3. Verbindungseinrichtung nach Anspruch 1 oder 2, **dadurch gekennzeichnet, dass** eine Aufstandsfläche (18) des Bodenrings (16) zumindest abschnittsweise konisch gestaltet ist, und dass der formstabile Bodenabschnitt (7) komplementär konisch gestaltet ist für eine flächige Abstützung des Bodenrings (16) auf dem Bodenabschnitt (7).

4. Verbindungseinrichtung nach einem der Ansprüche 1 bis 3, **dadurch gekennzeichnet, dass** eine Außenfläche (13) des deckelförmigen Kopfbereichs (12) eben gestaltet ist und bündig mit einer Randkante des Anschlussstutzens (3) abschließt.

5. Verbindungseinrichtung nach einem der Ansprüche 1 bis 4, **dadurch gekennzeichnet, dass** eine nach innen weisende Kontur (19) des deckelförmigen Kopfbereichs (12) domartig gestaltet ist.

6. Verbindungseinrichtung nach dem Oberbegriff des Anspruchs 1 oder nach einem der vorhergehenden Ansprüche, **dadurch gekennzeichnet, dass** der formstabile Bodenabschnitt (7a, 7c) eine ringförmige Gegenschulter (20, 20c) aufweist, die in den Bodenring (16a, 16c) des Ventilmantels (15a, 15c) in Richtung des Kopfbereichs (12a, 12c) hineinragt.

7. Verbindungseinrichtung nach Anspruch 6, **dadurch gekennzeichnet, dass** die Gegenschulter (20, 20c) eine ebene, radial zu einer Mittellängsachse (L) des Anschlussstutzens (3a, 3c) ausgerichtete Stirnfläche (21, 21c) aufweist.

8. Verbindungseinrichtung nach Anspruch 6 oder 7, **dadurch gekennzeichnet, dass** der Bodenabschnitt (7c) eine die Gegenschulter (20c) umgebende Ringnut (23) aufweist, in die der Ventilmantel (15c) bei einer elastischen Deformation teilweise eintaucht, und die in unbelastetem Ausgangszustand des Ventilkörpers (11c) einen freien Ringraum bildet.

9. Verbindungseinrichtung nach Anspruch 8, **dadurch gekennzeichnet, dass** die Ringnut (23) eine gestufte Ringprofilierung aufweist.

10. Verbindungseinrichtung nach dem Oberbegriff des Anspruchs 1 oder nach einem der vorhergehenden Ansprüche, **dadurch gekennzeichnet, dass** eine Innenkontur des Ventilmantels (15b) in Abstand zu einer Innenfläche des Kopfbereichs (12b) mit einer radial nach außen erstreckten Ringausnehmung (22) versehen ist, die insbesondere ein Festkörpergelenk für den Ventilmantel (15b) bei einer elastischen Deformation des Ventilkörpers (11b) definiert.

11. Verbindungseinrichtung nach Anspruch 10, **dadurch gekennzeichnet, dass** der Bodenring (16b) eine Ringschulter mit einem verdünnten Wandungsbereich aufweist, der derart gestaltet ist, dass bei einer axialen Druckbeanspruchung des Ventilkörpers (11b) vom Kopfbereich (12b) her ein axiales Einstülpen des Ventilmantels (15b) im Bereich des Bodenringes (16b) erfolgt.

12. Verbindungseinrichtung nach einem der vorhergehenden Ansprüche, **dadurch gekennzeichnet, dass** der Ventilmantel (15) rotationssymmetrisch und der Kopfbereich (12) rotationsunsymmetrisch, insbesondere oval, gestaltet ist.

13. Verbindungseinrichtung nach Anspruch 12, **dadurch gekennzeichnet, dass** die Schlitzanordnung (14) entlang einer Quererstreckung des Kopfbereichs (12) eingebracht ist.

14. Ventilkörper für eine Verbindungseinrichtung nach einem der vorhergehenden Ansprüche, der elastisch nachgiebig und becherartig gestaltet ist und mit einem Kopfbereich sowie einem Ventilmantel versehen ist, wie sie in den Merkmalen von wenigstens einem der vorhergehenden Ansprüche beschrieben sind

## Claims

1. Connecting device for a medical infusion system, having a connection piece (3) which has a connecting profile (9) for the connection of a functional part (F) of the infusion system, and having a resiliently flexible, cup-like valve body (11) which is arranged in the connection piece (3) and has a valve casing (15) and a cap-shaped top region (12) which is provided with a slit arrangement (14), and having a dimensionally stable base portion (7) on which a base ring (16) of the valve casing (15) is supported and which is firmly connected to the connection piece (3), **characterized in that** the valve casing (15), in the unloaded initial state, has a dished internal contour which, starting from the cap-shaped top region (12), first of all expands in the direction of the base ring (16) and subsequently narrows again, forming an O-like internal longitudinal section, wherein the valve casing (15) has a first wall portion that adjoins the top region (12), widens in a frustoconical manner and has a constant thickness, which first wall portion is adjoined, in the direction of the base ring (16), by a central wall portion, the inner wall of which extends cylindrically and coaxially with the longitudinal centre axis L, and the outer wall of which extends in a manner bulging further outwards in the direction of the base ring (16), and wherein the central wall portion is adjoined by a base-side wall portion, which comprises the base ring (16), and in which the inner wall tapers conically downwards from the central wall portion to the base portion.

2. Connecting device according to claim 1, **characterized in that** a wall of the valve casing (15) thickens from the top region (12) to the base ring (16).

3. Connecting device according to claim 1 or 2, **characterized in that** a contact area (18) of the base ring (16) is formed at least partly in a conical manner, and **in that** the dimensionally stable base portion (7) is formed in a complementary conical manner in order for the base ring (16) to be supported extensively on the base portion (7).

4. Connecting device according to one of Claims 1 to 3, **characterized in that** an outer face (13) of the cap-shaped top region (12) is formed in a planar manner and terminates flush with a peripheral edge of the connection piece (3).

5. Connecting device according to one of Claims 1 to 4, **characterized in that** an inwardly directed contour (19) of the cap-shaped top region (12) is formed in a domelike manner.

6. Connecting device according to the preamble of claim 1 or according to one of the preceding claims, **characterized in that** the dimensionally stable base portion (7a, 7c) has an annular mating shoulder (20, 20c) which projects into the base ring (16a, 16c) of the valve casing (15a, 15c) in the direction of the top region (12a, 12c).

7. Connecting device according to claim 6, **characterized in that** the mating shoulder (20, 20c) has a planar end face (21, 21c) oriented radially with respect to a longitudinal centre axis (L) of the connection piece (3a, 3c).

8. Connecting device according to claim 6 or 7, **characterized in that** the base portion (7c) has an annular groove (23) which surrounds the mating shoulder (20c), into which some of the valve casing (15c) protrudes in the event of elastic deformation, and which forms a free annular space in the unloaded initial state of the valve body (11c).

9. Connecting device according to claim 8, **characterized in that** the annular groove (23) has a stepped annular profile.

10. Connecting device according to the preamble of claim 1 or according to one of the preceding claims, **characterized in that** an internal contour of the valve casing (15b) is provided, at a distance from an inner face of the top region (12b), with a radially outwardly extended annular recess (22) which defines in particular a flexure bearing for the valve casing (15b) in the event of elastic deformation of the valve body (11b).

11. Connecting device according to claim 10, **characterized in that** the base ring (16b) has an annular shoulder with a thinned wall region which is formed such that, in the event of axial compressive stress being applied to the valve body (11b) from the top region (12b), the valve casing (15b) is axially inverted in the region of the base ring (16b).

12. Connecting device according to one of the preceding claims, **characterized in that** the valve casing (15) is formed in a rotationally symmetrical manner and the top region (12) is formed in a rotationally asymmetrical manner, in particular in an oval manner.

13. Connecting device according to claim 12, **characterized in that** the slit arrangement (14) has been introduced along a transverse extent of the top region (12).

14. Valve body for a connecting device according to one of the preceding claims, said valve body being formed in a resiliently flexible and cup-like manner and being provided with a top region and a valve casing, as are described in the features of at least one of the preceding claims.

## Revendications

1. Dispositif de liaison d'un système de perfusion médical, avec une tubulure de raccordement (3) qui comporte un profilage de liaison (9) servant à raccorder une partie fonctionnelle (F) du système de perfusion, ainsi qu'avec un corps de vanne (11) à souplesse élastique de type bécher qui est disposé dans la tubulure de raccordement (3) et comporte une enveloppe de vanne (15) ainsi qu'une région de tête en forme de cache (12) pourvue d'un agencement de fente (14) et avec une section de plancher de forme stable (7) sur laquelle une bague de plancher (16) de l'enveloppe de vanne (15) est soutenue et qui est reliée fixement à la tubulure de raccordement (3), **caractérisé en ce que** l'enveloppe de vanne (15) présente dans l'état de sortie non sollicité un contour intérieur bombé s'élargissant d'abord à partir de la région de tête en forme de cache (12) en direction de la bague de plancher (16) et se rétrécissant ensuite de nouveau en formant une découpe longitudinale intérieure en forme de O, l'enveloppe de vanne (15) comportant une première section de paroi d'épaisseur constante raccordée au niveau de la région de tête (12), s'élargissant à la façon d'un tronc de cône et au niveau de laquelle une section de paroi centrale est raccordée en direction de la bague de plancher (16), la paroi intérieure de ladite section s'étendant de façon cylindrique et dans le plan coaxial par rapport à un axe longitudinal central L et la paroi extérieure de ladite section s'étendant de façon bombée vers l'extérieur, davantage en direction de la bague de plancher (16), et une section de paroi du côté de plancher étant raccordée au niveau de la section de paroi centrale, ladite section de paroi comprenant la bague de plancher (16) et la paroi intérieure dans laquelle elle se trouve s'étendant de façon à se rétrécir vers le bas en cône depuis la section de paroi centrale, en direction de la section de plancher.

2. Dispositif de liaison selon la revendication 1, **caractérisé en ce qu'**une paroi de l'enveloppe de vanne (15) s'épaissit depuis la région de tête (12) jusque vers la bague de plancher (16).

3. Dispositif de liaison selon la revendication 1 ou 2, **caractérisé en ce qu'**une surface d'appui (18) de la bague de plancher (16) est réalisée au moins en partie de façon conique et que la section de plancher de forme stable (7) est réalisée de façon conique de façon complémentaire, pour un appui plat de la bague de plancher (16) sur la section de plancher (7).

4. Dispositif de liaison selon l'une quelconque des revendications 1 à 3, **caractérisé en ce qu'**une surface extérieure (13) de la région de tête en forme de cache (12) est agencée de façon plane et se termine en affleurement par une arête de bordure de la tubulure de raccordement (3).

5. Dispositif de liaison selon l'une quelconque des revendications 1 à 4, **caractérisé en ce qu'**un contour (19), orienté vers l'intérieur, de la région de tête en forme de cache (12) est réalisé à la façon d'un dôme.

6. Dispositif de liaison selon le préambule de la revendication 1 ou selon l'une quelconque des revendications précédentes, **caractérisé en ce que** la section de plancher de forme stable (7a, 7c) comporte un contre-épaulement (20, 20c) de forme annulaire rentrant dans la bague de plancher (16a, 16c) de l'enveloppe de vanne (15a, 15c) en direction de la région de tête (12a, 12c).

7. Dispositif de liaison selon la revendication 6, **caractérisé en ce que** le contre-épaulement (20, 20c) comporte une surface avant (21, 21c) plane orientée dans le plan radial par rapport à un axe longitudinal central (L) de la tubulure de raccordement (3a, 3c).

8. Dispositif de liaison selon la revendication 6 ou 7, **caractérisé en ce que** la section de plancher (7c) comporte une rainure annulaire (23) entourant le contre-épaulement (20c) dans laquelle l'enveloppe de vanne (15c) plonge en partie en présence d'une déformation élastique et qui forme un espace annulaire libre dans l'état de sortie non sollicité du corps de vanne (11c).

9. Dispositif de liaison selon la revendication 8, **caractérisé en ce que** la rainure annulaire (23) présente un profilage annulaire étagé.

10. Dispositif de liaison selon le préambule de la revendication 1 ou selon l'une quelconque des revendications précédentes, **caractérisé en ce qu'**un contour intérieur de l'enveloppe de vanne (15b) est pourvu d'un évidement annulaire (22) s'étendant dans le plan radial vers l'extérieur à une certaine distance de la surface intérieure de la région de tête (12b) qui définit notamment une articulation monolithique pour l'enveloppe de vanne (15b) en cas de déformation élastique du corps de vanne (11b).

11. Dispositif de liaison selon la revendication 10, **caractérisé en ce que** la bague de plancher (16b) comporte un épaulement annulaire avec une région de paroi amincie réalisée de telle sorte qu'en cas de sollicitation en compression axiale du corps de vanne (11b) à partir de la région de tête (12b), un retournement axial de l'enveloppe de vanne (15b) se produit dans la région de la bague de plancher (16b).

12. Dispositif de liaison selon l'une quelconque des revendications précédentes, **caractérisé en ce que** l'enveloppe de vanne (15) est agencée symétriquement en rotation et que la région de tête (12) est réalisée asymétriquement en rotation, notamment de façon ovale.

13. Dispositif de liaison selon la revendication 12, **caractérisé en ce que** l'agencement de fente (14) est amené le long d'un prolongement transversal de la région de tête (12).

14. Corps de vanne pour un dispositif de liaison selon l'une quelconque des revendications précédentes qui est agencé à souplesse élastique de type bécher et est pourvu d'une région de tête ainsi que d'une enveloppe de vanne, tel que décrit dans les caractéristiques d'au moins une des revendications précédentes.
